Europäisches Patentamt

**European Patent Office**

**Office européen des brevets**

(11) Numéro de publication: **0 107 586**
**A1**

## DEMANDE DE BREVET EUROPEEN

(21) Numéro de dépôt: 83402018.2

(22) Date de dépôt: 18.10.83

(51) Int. Cl.³: **A 23 K 1/175**, A 61 K 33/00,
**A 23 L 1/30**, A 61 K 9/00

(30) Priorité: 21.10.82 FR 8217606

(43) Date de publication de la demande: 02.05.84
**Bulletin 84/18**

(84) Etats contractants désignés: **AT BE CH DE GB IT LI LU NL SE**

(71) Demandeur: **Moldauer, Yves, 35, avenue Sainte Foy, F-92200 Neuilly (FR)**

(72) Inventeur: **Moldauer, Yves, 35, avenue Sainte Foy, F-92200 Neuilly (FR)**

(74) Mandataire: **Tanguy, Gilbert André et al, 10-12, rue Rosenwald, F-75015 Paris (FR)**

(54) **Oligo-éléments électro-négatifs, composition les renfermant et procédé pour leur préparation.**

(57) L'invention concerne les oligo-éléments électro-négatifs.
Ces derniers sont obtenus par un procédé selon lequel on capte des ions électro-négatifs produits par un générateur approprié, et l'on met en contact ces ions électro-négatifs avec un matériau, par exemple une masse métallique, susceptible de produire des oligo-éléments qui, en présence des ions électro-négatifs captés, sont transformés en oligo-éléments électro-negatifs.

EP 0 107 586 A1

OLIGO-ELEMENTS ELECTRO-NEGATIFS, COMPOSITION
LES RENFERMANT ET PROCEDE POUR LEUR PREPARATION.

La présente invention est du domaine des compléments alimentaires. Elle concerne plus précisément des compléments alimentaires, adjuvants et médicaments visant à un rétablissement d'un équilibre ionique favorable dans un milieu ou un organe physiologique.

On connaît jusqu'à présent les bienfaits qu'apportent les traitements par ions négatifs sur le rétablissement d'un équilibre ionique favorable et sur le comportement humain, animal et végétal, en général. On peut trouver dans le commerce un certain nombre d'appareils générateurs d'ions électro-négatifs, ces appareils étant notamment destinés à rétablir dans l'environnement immédiat d'un sujet l'équilibre ionique le plus favorable. Malheureusement, ces ions électro-négatifs ont une durée de vie relativement courte, du fait de leur destruction par les différents éléments chargés d'électricité positive, et partant leur action bénéfique est de courte durée. Cet inconvénient ne permet donc pas le transfert d'ions électro-négatifs sur de longues distances pendant des périodes de temps relativement importantes et, jusqu'à présent, il n'a pas été possible de résoudre le problème du transport de tels ions.

La présente invention a pour objet, à titre de produits industriels nouveaux, les oligo-éléments électro-négatifs, grâce auxquels on peut continuer à tirer bénéfice des propriétés favorables des ions électro-négatifs par leur association avec des oligo-éléments, pour former lesdits oligo-éléments électro-négatifs.

Un autre objet de l'invention est de permettre le transport de ces oligo-éléments électro-négatifs à distance sur un support approprié, physiologiquement compatible pour être assimilé par un organisme humain, animal ou végétal sous la forme d'un complément alimentaire, d'un adjuvant et d'un médicament.

Un troisième objet de l'invention est de fournir un procédé de production des oligo-éléments électro-négatifs de l'invention, et de les conditionner sous la forme de compléments alimentaires, d'adjuvants ou de médicaments.

L'invention vise donc à fournir des compléments alimentaires, caractérisés par le fait qu'ils sont constitués par des oligo-éléments, autres que les oligo-éléments électro-positifs, avantageusement des oligo-éléments électro-négatifs, éventuellement associés à un support physiologiquement compatible, pratiquement exempt ou rendu exempt d'éléments électro-positifs.

Selon un mode de réalisation préféré, le support des oligo-éléments électro-négatifs est constitué par de l'eau exempte ou rendue exempte d'éléments électro-positifs.

La présente invention concerne également un procédé de préparation d'oligo-éléments électro-négatifs, caractérisé par le fait que l'on capte des ions électro-négatifs produits par un générateur approprié, et que l'on met en contact ces ions électro-négatifs avec un matériau susceptible de produire des oligo-éléments qui, mis en présence des ions électro-négatifs produits par ledit générateur, sont transformés en oligo-éléments électro-négatifs.

Selon un mode de mise en oeuvre préféré de ce procédé, le matériau susceptible de produire des oligo-éléments est un ou plusieurs métaux, les oligo-éléments obtenus par le procédé décrit ci-dessus sont transférés dans un milieu physiologiquement compatible pratiquement exempt ou rendu exempt d'éléments électro-positifs, par exemple de l'eau déminéralisée, chimiquement pure ou très faiblement minéralisée.

L'invention se rapporte également à un dispositif pour la mise en oeuvre du procédé de préparation d'oligo-éléments électro-négatifs selon l'invention, ce dispositif étant caractérisé par le fait qu'il comporte :

    - un générateur d'ions électro-négatifs;

    - un capteur de ces ions électro-négatifs;

    - un matériau recevant dudit capteur ces ions électro-négatifs, et capable de fournir des oligo-éléments susceptibles de se combiner avec lesdits ions électro-négatifs captés pour fournir des oligo-éléments électro-négatifs de l'invention.

Ce dispositif comporte également un agencement de conditionnement de ces oligo-éléments électro-négatifs sur un support ou milieu physiologiquement compatible, pratiquement exempt ou rendu exempt d'éléments électro-positifs.

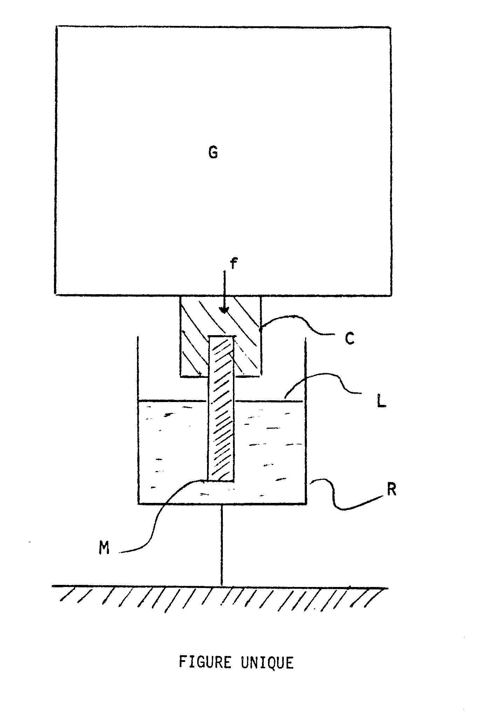

L'invention sera mieux comprise à la lecture de la description non limitative suivante de formes de réalisation préférées de l'invention, en référence au dessin annexé, dans lequel la figure unique représente un schéma bloc d'une forme de réalisation particulière d'un dispositif de production d'oligo-éléments électro-négatifs selon l'invention.

Sur le dessin annexé est représenté un générateur G d'électrons électro-négatifs, qu'on peut se procurer dans le commerce, par exemple un générateur à effet de pointe fabriqué et commercialisé par le laboratoire de Born. Ce générateur G est connecté dans la direction privilégiée, selon la flèche _f_, de production des ions électro-négatifs, à un capteur C présentant des caractéristiques de conductibilité favorables au transfert des ions électro-négatifs. Ce capteur C est lui-même raccordé à un matériau, par exemple une masse métallique M, en contact conducteur par au moins une de ses faces audit capteur C. Par ailleurs, une partie de cette masse M est plongée dans un milieu exempt ou rendu exempt d'éléments électro-positifs, lui-même contenu dans un récipient relié à la terre. Comme milieu exempt d'éléments électro-positifs, on utilise de préférence de l'eau chimiquement pure, déminéralisée, ou très faiblement minéralisée.

Le dispositif de production d'oligo-éléments électro-négatifs selon l'invention fonctionne de la manière suivante :

On commence par connecter le générateur à une source de puissance, par exemple un courant électrique, de façon à obtenir un flux d'ions électro-négatifs selon la flèche _f_ vers le capteur C, qui transfère ces ions électro-négatifs à la masse métallique M dans laquelle ils se combinent avec des oligo-éléments générés par cette masse M, qui deviennent des oligo-éléments électro-négatifs qui migrent à leur tour dans le liquide L, par exemple de l'eau, contenu dans le récipient R. Le liquide L peut, après une certaine période de fonctionnement du générateur G, par exemple de l'ordre de plusieurs heures, être prélevé et administré comme adjuvant, complément alimentaire ou médicament, à des organismes humains, animaux ou végétaux. Par exemple, lorsque la masse métallique M est constituée par du magnésium, on obtient, après un fonctionnement de l'appareil de 45 h, un complément alimentaire renfermant 45 mg de magnésium par litre de support, en l'occurrence de l'eau.

Il est clair que l'invention n'est nullement limitée à la forme de réalisation décrite ci-dessus, en référence au dessin annexé, mais qu'elle englobe toutes les modifications et variantes issues du même principe de base, c'est-à-dire la production d'oligo-éléments électronégatifs à partir d'ions électro-négatifs. C'est ainsi que les oligo-éléments électro-négatifs de l'invention peuvent être utilisés comme médicaments ou adjuvants dans des compositions destinées à agir sur le métabolisme des êtres vivants.

4

Selon une variante le dispositif de production des oligo-elements électro-négatifs selon l'invention, peut être simplifié en connectant directement le générateur d'ions électro-négatifs au matériau, par exemple une masse métallique, capable de fournir des oligo-éléments susceptibles de se combiner avec les ions électro-négatifs captés pour fournir des oligo-éléments électro-négatifs.

0107586

5

REVENDICATIONS

1. Compléments alimentaires, caractérisés par le fait qu'ils sont constitués par des oligo-éléments, à l'exception des oligo-éléments électro-positifs, éventuellement associés à un support physiologiquement compatible, pratiquement exempt ou rendu exempt d'éléments électro-positifs.

2. A titre de produits industriels nouveaux, les oligo-éléments électro-négatifs.

3. Compléments alimentaires, caractérisés en ce qu'ils sont constitués par des oligo-éléments électro-négatifs, éventuellement associés à un support physiologiquement compatible, pratiquement exempt ou rendu exempt d'éléments électro-positifs.

4. Compléments alimentaires selon la revendication 3, caractérisés en ce que le support des oligo-éléments électro-négatifs est constitué par de l'eau chimiquement pure, déminéralisée ou très faiblement minéralisée.

5. Procédé de préparation d'oligo-éléments électro-négatifs, caractérisé par le fait que l'on capte des ions électro-négatifs produits par un générateur approprié, et que l'on met en contact ces ions électro-négatifs avec un matériau, par exemple une masse métallique, susceptible de produire des oligo-éléments qui, en présence des ions électro-négatifs captés, sont transformés en oligo-éléments électro-négatifs.

6. Procédé selon la revendication 5, caractérisé par le fait que les oligo-éléments électro-négatifs ainsi produits sont transférés dans un support ou milieu physiologiquement compatible, pratiquement exempt ou rendu exempt d'éléments électro-positifs, par exemple de l'eau chimiquement pure ou déminéralisée, ou très faiblement minéralisée.

7. Dispositif pour la production d'oligo-éléments électro-négatifs, caractérisé en ce qu'il comporte :

- un générateur d'ions électro-négatifs;

- un capteur de ces ions électro-négatifs produits par le générateur;

- un matériau, par exemple une masse métallique, connecté à ce capteur et capable de fournir des oligo-éléments susceptibles de se combiner avec les ions électro-négatifs captés pour fournir des oligo-éléments électro-négatifs.

6

8. Dispositif selon la revendication 7, caractérisé en ce qu'est prévu un agencement renfermant un support ou milieu physiologiquement compatible dans lequel les oligo-éléments électro-négatifs peuvent être conditionnés.

9. Les compositions médicamenteuses, caractérisées en ce qu'elles renferment des oligo-éléments, à l'exception des oligo-éléments électro-positifš, seuls ou associés à au moins un support physiologiquement compatible.

10. Les compositions médicamenteuses, caractérisées en ce qu' elles renferment des oligo-éléments électro-négatifs, seuls ou associés à au moins un support physiologiquement compatible.

11. Dispositif pour la production d'oligo-éléments électro-négatifs, caractérisé par le fait qu'il comporte :

- un générateur d'ions électro-négatifs;
- un matériau recevant dudit générateur ces ions électro-négatifs et capable de fournir des oligo-éléments susceptibles de se combiner avec lesdits ions électro-négatifs captés pour fournir des électro-éléments électro-négatifs désirés.

FIGURE UNIQUE

0107586

**Office européen des brevets**

**RAPPORT DE RECHERCHE EUROPEENNE**

Numéro de la demande

EP  83 40 2018

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl. 3) |
|---|---|---|---|
| X | FR-A- 977 749 (A.O. DEMOLON et E.M. BASTISSE) <br> * page 1, alinéas 6,8; page 2, alinéa 4 * | 2,9,10 | A 23 K 1/175 <br> A 61 K 33/00 <br> A 23 L 1/30 <br> A 61 K 9/00 |
| | --- | | |
| Y | FR-A-1 280 562 (G.G.H. BAER) <br> * en entier * | 1-11 | |
| | --- | | |
| Y | FR-A-1 142 722 (M. VIOLET) <br> * en entier, en particulier page 1, alinéas 2,8 * | 1-11 | |
| | --- | | |
| A | FR-A-1 603 804 (W. SEUSS) | | |
| | --- | | |
| A | FR-A-2 191 934 (PILLIOT-PINARDEL J.P.) | | DOMAINES TECHNIQUES RECHERCHES (Int. Cl. 3) |
| | --- | | |
| A | FR-M- 131 (R.L.A. VALTAT) | | A 23 K <br> A 23 L <br> A 61 K <br> C 05 D |
| | ----- | | |

Le présent rapport de recherche a été établi pour toutes les revendications

| Lieu de la recherche | Date d'achèvement de la recherche | Examinateur |
|---|---|---|
| LA HAYE | 02-12-1983 | GALLIGANI L. |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

& : membre de la même famille, document correspondant

OEB Form 1503 03 82